(19) Europäisches Patentamt
European Patent Office
Office européen des brevets

(11) Publication number: 0 379 935 A1

(12) # EUROPEAN PATENT APPLICATION

(21) Application number: 90100832.6

(22) Date of filing: 16.01.90

(51) Int. Cl.5: C07C 69/94, C07D 317/60, C07C 67/347, C07C 47/575, C07D 317/54, C07C 69/157

Claims for the following Contracting States: ES + GR

(30) Priority: 27.01.89 JP 18587/89

(43) Date of publication of application: 01.08.90 Bulletin 90/31

(84) Designated Contracting States: AT BE CH DE DK ES FR GB GR IT LI LU NL SE

(71) Applicant: TANABE SEIYAKU CO., LTD.
2-10, Dosho-machi 3-chome
Chuo-ku Osaka(JP)

(72) Inventor: Iwasaki, Tameo
No. 27-14-411, Hinoike-cho
Nishinomiya-shi, Hyogo-ken(JP)
Inventor: Ohmizu, Hiroshi
No. 2-308 Mibubojo-dainidanchi, 48-3, Mibubojo-cho
Nakagyo-ku, Kyoto-shi, Kyoto-fu(JP)
Inventor: Tsuyoshi, Ohgiku
No. 6-3, Mondohigashi-machi
Nishinomiya-shi, Hyogo-ken(JP)

(74) Representative: Hansen, Bernd, Dr.rer.nat. et al
Hoffmann, Eitle & Partner Patentanwälte
Arabellastrasse 4 Postfach 81 04 20
D-8000 München 81(DE)

(54) **A process for preparing naphthalene derivatives.**

(57) A novel process for preparing a naphthalene derivative of the formula:

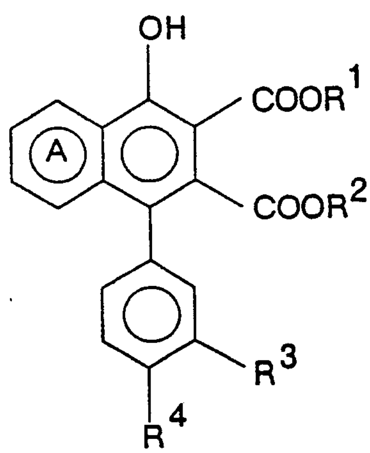

(I)

wherein each of $R^1$ and $R^2$ is a lower alkyl group; one of $R^3$ and $R^4$ is hydrogen atom or a lower alkoxy group and the other is a lower alkoxy group; and Ring A is a substituted or unsubstituted benzene ring, or a pharmaceutically acceptable salt thereof is disclosed. Said compound is useful as a hypolipidemic agent.

EP 0 379 935 A1

## A PROCESS FOR PREPARING NAPHTHALENE DERIVATIVES

This invention relates to a novel process for preparing a naphthalene derivative having excellent hypolipidemic activities and an intermediate thereof.

It is known that 1-(3- and/or 4-lower alkoxy phenyl)-2,3-bis(lower alkoxycarbonyl)-4-hydroxynaphthalene derivatives and the salt thereof are useful hypolipidemic agents. It is also known that said naphthalene derivatives can be prepared by reacting a di-lower alkylacetal of 2-(α-hydroxy-3 and/or 4-lower alkoxybenzyl)benzaldehyde with a lower alkyl acetylenedicarboxylate [Japanese Patent Publication (unexamined) No. 267541/1986].

The object of the present invention is to provide a novel and economical process for industrial scale production of the naphthalene derivatives.

According to the present invention, a naphthalene derivative of the formula:

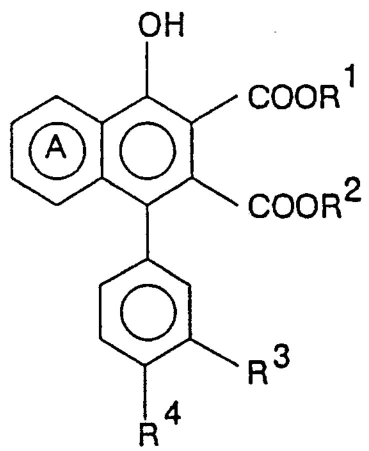

(I)

wherein each of $R^1$ and $R^2$ is a lower alkyl group; one of $R^3$ and R4 is hydrogen atom or a lower alkoxy group and the other is a lower alkoxy group; and Ring A is a substituted or unsubstituted benzene ring, can be prepared by reacting an α-protected hydroxy aldehyde compound of the formula:

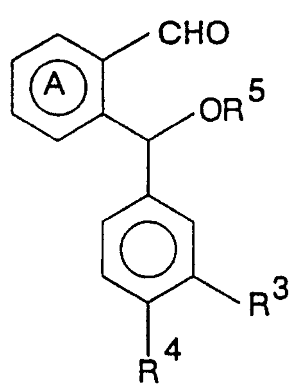

(II)

wherein $OR^5$ is a protected hydroxy group, and $R^3$, $R^4$ and Ring A are the same as defined above, or its di-lower alkylacetal with a lower alkyl acetylenedicarboxylate of the formula:

O O<br>
|| ||<br>
$R^1$-O C-C≡C- C O-$R^2$ (III)

wherein $R^1$ and $R^2$ are the same as defined above.

The reaction is accomplished preferably in the presence of an acid. Examples of the acid include organic and inorganic acids such as lower alkanoic acids (e.g., formic acid, acetic acid), trihalo-lower alkanoic acids (e.g., trifluoroacetic acid), benzenesulfonic acid, (lower alkylbenzene)sulfonic acids (e.g., p-toluenesulfonic acid), lower alkylsulfonic acids (e.g., methanesulfonic acid, ethanesulfonic acid), mineral

acids (e.g., hydrochloric acid, hydrobromic acid, sulfuric acid, nitric acid, phosphoric acid), stannic halides (e.g., stannic chloride), aluminum halides (e.g., aluminum trichloride), titanium halides (e.g., titanium tetrachloride), zinc halides (e.g., zinc chloride), ferric halides (e.g., ferric chloride), boron halides-etherate (e.g., boron trichloride-etherate) and the like. Among them, organic acids are preferably used in the invention. The protected hydroxy group include any of those which can be eliminated as a protected-oxy anion. Examples of such protected hydroxy group include hydroxy group protected by a lower alkyl group, a lower alkanoyl group and the like. The reaction may be carried out either in a solvent or without a solvent. Examples of the solvents include ethereal solvents (e.g., tetrahydrofuran, ether, dioxane), halogenated hydrocarbons (e.g., methylenechloride, chloroform) and aromatic solvents (e.g., benzene, toluene, xylene). The reaction may be carried out at room temperature or under heating, preferably at a temperature between 50° C and 100° C.

According to the process of the present invention, various naphthalene derivatives as disclosed in the Japanese Patent Publication (unexamined) No. 267541/ 1986 can be prepared in high yields. Representative examples of such naphthalene derivatives include those of the formula (I) wherein Ring A is unsubstituted benzene ring, a benzene ring substituted with a lower alkylenedioxy group or a benzene ring having one to 3 substituent(s) selected from a group consisting of a lower alkoxy group, a lower alkyl group, a phenyl-lower alkoxy group, hydroxy group and a halogen atom.

Such naphthalene derivatives include as subgenera those of the formula (I) wherein Ring A is a benzene ring of the formula:

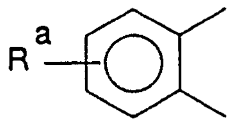 , 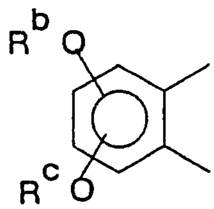 , 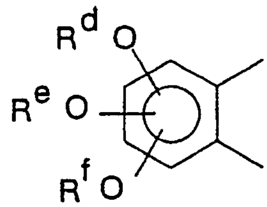

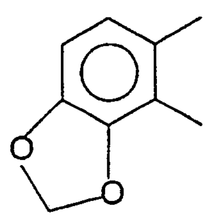 , 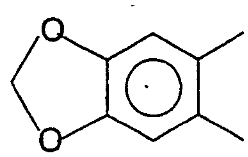 or 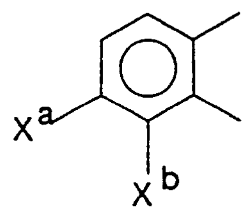

wherein $R^a$ is hydrogen atom, a lower alkyl group or a lower alkoxy group, $R^b$ is a lower alkyl group, $R^c$ is a lower alkyl group, a phenyl-lower alkyl group or hydrogen atom, each of $R^d$, $R^e$ and $R^f$ is a lower alkyl group and each of $X^a$ and $X^b$ is a halogen atom, and those of the formula (I) wherein each of $R^3$ and $R^4$ is a lower alkoxy group and Ring A is a benzene ring of the formula:

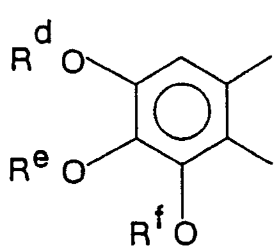

wherein $R^d$, $R^e$ and $R^f$ are the same as defined above.

The compound (I) thus-obtained may be, if required, further converted into an alkali metal salt, an alkaline earth metal salt or a quarternally ammonium salt thereof by a conventional method.

It has been generally recognized that, in carrying out the reaction of the di-lower alkylacetal of 2-benzylbenzaldehyde or its di-lower alkylacetal with an acetylenedicarboxylate, said benzaldehyde must always have a free hydroxy group at the α-position of the benzyl moiety. For example, H. P. Paulmann et al reported that the reaction between 2-[α-hydroxy-3,4-dimethoxybenzyl]-4,5-methylenedioxybenzaldehyde dimethyl acetal and dimethyl acetylenedicarboxylate proceeds via the steps involving ① intramolecular

cyclization of said benzaldehyde dimethyl acetal between the hydroxy and the acetal groups thereof, followed by ② elimination of one methanol molecule therefrom to give isobenzofuran intermediate, which undergoes ③ Diels-Alder reaction with dimethyl acetylenedicarboxylate [J. Chem. Soc. Chem. Comm. 354 - 355(1980)].

In contrast to the method of H. P. Paulmann et al, however, the process of the present invention is accomplished by the use of the starting compound (II) which has the protected hydroxy group at the α-position of the benzyl moiety. This indicates that the process of the present invention is quite different in its reaction mechanism from that of Japanese Patent Publication (unexamined) No. 26754/1986. In addition, as compared with the known processes, the process of the present invention is more advantageous in that the compound (I) can be prepared in a high yield without unfavorable by-products, and also that the starting compound (II) or its di-lower alkyl acetal is accompanied with no substantial polimerization due to the presence of a free hydroxy group during the reaction and/or storage thereof.

Further, the starting compound (II) wherein $OR^5$ is a lower alkanoyloxy group can be readily obtained as crystals by simple recrystallization from an ordinary solvent (e.g., isopropylether) and used efficiently for the reaction with the acetylenedicarboxylate compound (III), whereas the di-lower alkyl acetal of unprotected α-hydroxybenzyl-benzaldehyde compound is usually obtained as an oil and requires additional purification.

Concomitantly, the aldehyde compound (II) or its di-lower alkyl acetal used as the starting compound of the present invention may be obtained easily by reacting the corresponding α-hydroxybenzy-benzaldehyde compound or its di-lower alkyl acetal with a lower alkyl halide, a reactive derivative of a lower alkanoic acid (e.g., active esters, halides and acid anhydrides of a lower alkanoic acid) and the like in the presence of an acid acceptor according to a conventional method.

Throughout the specification and claims, the terms "lower alkyl", "lower alkoxy", "lower alkanoyl" and "lower alkylenedioxy" should be interpreted as referring to alkyl of one to 6 carbon atoms and alkoxy of one to 6 carbon atoms, alkanoyl of 2 to 7 carbon atoms and alkylenedioxy of one to 4 carbon atoms, especially alkyl of one to 4 carbon atoms and alkoxy of one to 4 carbon atoms, alkanoyl of 2 to 5 carbon atoms and alkylenedioxy of one or 2 carbon atoms.

Example 1

(1) 5.0 g of 2-(α-hydroxy-3,4-dimethoxybenzyl)-3,4,5-trimethoxybenzaldehyde dimethylacetal are dissolved in 50 ml of methylenechloride. 3.8 ml of triethylamine, 3.0 ml of acetic anhydride and 100 mg of 4-dimethylaminopyridine are added to the solution. And the mixture is stirred at room temperature for 3 days. The reaction mixture is washed, dried and evaporated to remove the solvent, whereby 5.2 g of 2-(α-acetoxy-3,4-dimethoxybenzyl)-3,4,5-trimethoxybenzaldehyde dimethylacetal are obtained.
NMR ($CDCl_3$, δ): 2.17 (s, 3H), 3.11 (s, 3H), 3.47 (s, 3H), 3.74 (s, 3H), 3.79 (s, 3H), 3.83 (s, 3H), 3.86 (s, 3H), 3.88 (s, 3H), 5.53 (s, 1H), 6.55 - 6.95 (m, 3H), 7.00 (s, 1H), 7.38 (s, 1H)

(2) 5.2 g of the product obtained in paragraph (1) are dissolved in a mixture of 100 ml of tetrahydrofuran and 20 ml of water. 9 ml of 10 % hydrochloric acid are added thereto under ice-cooling, and the mixture is stirred at the same temperature for 2 hours. Chilled water is added to the reaction mixture, and the mixture is extracted with ethyl acetate. The extract is washed, dried and evaporated to remove the solvent. The resulting residue is purified by silica gel column chromatography [solvent: toluene-ethyl acetate (5: 1)] and recrystallized from isopropyl ether, whereby 4.27 g of 2-(α-acetoxy-3,4-dimethoxybenzyl)-3,4,5-trimethoxybenzaldehyde are obtained as colorless crystals.
M.p. 96 - 98 °C

$$IR\ \nu_{Max}^{Nujol}\ (cm^{-1}):\ 1740,\ 1680$$

(3-a) 780 mg of the product obtained in paragraph (2) are dissolved in 20 ml of benzene. 0.7 ml of dimethyl acetylenedicarboxylate and 5 ml of trifluoroacetic acid are added thereto. The mixture is stirred at 60 °C for 4 hours, and evaporated to remove the solvent. Methanol is added to the resulting residue, and the crystalline precipitates are collected by filtration, whereby 750 mg of 1-(3,4-dimethoxyphenyl)-2,3-bis-(methoxycarbonyl)-4-hydroxy-6,7,8-trimethoxynaphthalene are obtained as colorless prisms. Yield: 77%
M.p. 178-179 °C

(3-b) The product obtained in paragraph (1) is reacted with dimethyl acetylenedicarboxylate in the same manner as described in paragraph (3-a), whereby 1-(3,4-dimethoxyphenyl)-2,3-bis(methoxycarbonyl)-4-hydroxy-6,7,8-trimethoxynaphthalene is obtained.

Thus-obtained product has the same physico-chemical properties as the product of paragraph (3-a).

(4) A solution of 4.86 g of the product obtained in paragraph (3-a) or (3-b) in 100 ml of tetrahydrofuran is added to a solution of 0.387 g of 62.5 % sodium hydride in 10 ml of tetrahydrofuran at room temperature under stirring. The mixture is stirred at the same temperature for one hour and evaporated under reduced pressure at a temperature below 30 °C. The resulting residue is triturated with petroleum ether, whereby 4.8 g of sodium salt of 1-(3,4-dimethoxyphenyl)-2,3-bis(methoxycarbonyl)-4-hydroxy-6,7,8-trimethoxynaphthalene are obtained as powder.

$$IR\ \nu^{Nujol}_{Max}\ (cm^{-1}): 1710, 1680, 1600$$

Example 2

(1) 0.93 g of 2-(α-hydroxy-3,4-dimethoxybenzyl)-3,4,5-trimethoxybenzaldehyde dimethylacetal are dissolved in 15 ml of tetrahydrofuran. A suspension of 65 mg of sodium hydride in 10 ml of dimethylformamide is added dropwise thereto, and the mixture is stirred at room temperature for 3 hours. After the reaction mixture is ice-cooled, 0.17 ml of methyl iodide is added thereto. The mixture is stirred at room temperature for 16 hours and evaporated to remove the solvent. The resulting residue is poured into a mixture of an aqueous saturated sodium bicarbonate solution and diethyl ether, and the organic layer is obtained. The aqueous layer is further extracted with diethyl ether, and the extract is combined with the organic layer, washed, dried and evaporated to remove the solvent, whereby 0.87 g of 2-(α-methoxy-3,4-dimethoxybenzyl)-3,4,5-trimethoxybenzaldehyde dimethylacetal is obtained.

NMR ($CDCl_3$, δ): 2.91 (s, 3H), 3.40 (s, 6H), 3.77 (s, 3H), 3.84 (s, 6H), 3.89 (s, 6H), 5.43 (s, 1H), 5.90 (s, 1H), 6.65 - 7.05 (m,3H)

(2) 0.87 g of the product obtained in paragraph (1) is dissolved in 30 ml of tetrahydrofuran. 20 ml of 0.001 M hydrochloric acid are added thereto and the mixture is stirred at room temperature for 30 minutes. The reaction mixture is evaporated to remove the solvent, and the resulting residue is shaken with a mixture of ethyl acetate and water. The organic layer is obtained, washed, dried and evaporated to remove the solvent. The residue is purified by silica gel column chromatography [solvent: toluene-ethyl acetate (10: 1)], and the eluate is evaporated to remove the solvent, whereby 680 mg of 2-(α-methoxy-3,4-dimethoxybenzyl)-3,4,5-trimethoxy-benzaldehyde are obtained.

NMR ($CDCl_3$, δ): 3.39 (s, 3M), 3.82 (s, 3H) 3.83 (s, 3H), 3.86 (s, 3H), 3.91 (s, 3H), 3.97 (s, 3H), 5.99 (s, 1H), 6.55 - 7.05 (m, 3H), 7.37 (s, 1H), 10.32 (s, 1H)

(3) 220 mg of the product obtained in paragraph (2) are dissolved in 10 ml of benzene. 0.11 ml of dimethyl acetylenedicarboxylate and 0.002 ml of trifluoroacetic acid are added thereto, and the mixture is refluxed for 30 minutes. The reaction mixture is evaporated to remove the solvent, and methanol is added to the resulting residue. The crystalline precipitates are collected by filtration, whereby 160 mg of 1-(3,4-dimethoxyphenyl)-2,3-bis(methoxycarbonyl)-4-hydroxy-6,7,8-trimethoxynaphthalene are obtained as colorless prisms.

The filtrate is purified by silica gel column chromatography [solvent: ethyl acetate-toluene], and the eluate is evaporated to remove the solvent, whereby 40 mg of 1-(3,4-dimethoxyphenyl)-2,3-bis-(methoxycarbonyl)-4-hydroxy-6,7,8-trimethoxynaphthalene are further obtained. Yield: 70 %

Thus-obtained product has the same physico-chemical properties as the product of Example 1-(3-a).

Examples 3 and 4

(1) The corresponding starting compounds are treated in the same manner as described in Example 2-(1) and (2), whereby the compounds listed in the following Table 1 are obtained.

## Table 1

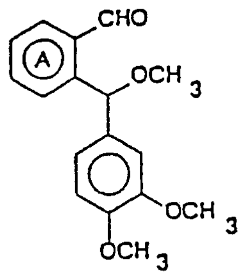

| Example Nos. | Ring A | Physical properties etc. |
|---|---|---|
| 3-(1) | 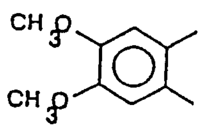 | NMR ($CDCl_3$, δ): 3.40 (s, 3H), 3.81 (s, 3H), 3.84 (s, 3H), 3.90 (s, 3H), 3.94 (s, 3H), 6.04 (s, 1H), 6.77 - 6.85 (m, 2H), 6.93 - 7.02 (m, 1H), 7.14 (s, 1H), 7.37 (s, 1H), 10.15 (s, 1H)<br>Yield: 70 % |
| 4-(1) | 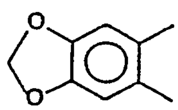 | NMR ($CDCl_3$, δ): 3.38 (s, 3H), 3.82 (s, 3H), 3.84 (s, 3H), 6.00 - 6.10 (m, 3H), 6.80 - 6.87 (m, 2H), 6.88 - 6.98 (m, 1H), 7.05 (s, 1H), 7.29 (s, 1H), 10.14 (s, 1H)<br>Yield: 72 % |

(2) The products obtained in paragraph (1) are treated in the same manner as described in Example 2-(3), whereby the compounds listed in the following Table 2 are obtained.

## Table 2

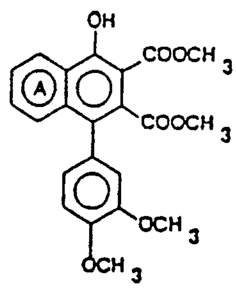

| Example Nos. | Ring A | Physical properties etc. |
|---|---|---|
| 3-(2) | 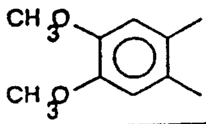 | M.p. 208 - 209 °C<br>Yield: 77% |
| 4-(2) | 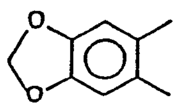 | M.p. 130 - 133 °C<br>Yield: 72 % |

Examples 5-21

The corresponding starting compounds are treated in the same manner, as described in either one of Examples 1 & 2, whereby the compounds listed in the following Tables 3 & 4 are obtained.

Table 3

| Example Nos. | 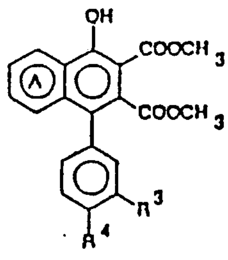 | | |
|---|---|---|---|
| | Ring A | $R^3$ / $R^4$ | Physical properties etc. |
| 5 | 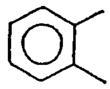 | $R^3$ =-OCH3<br>$R^4$ =-OCH3 | Colorless crystals<br>M.p. 182 - 184 °C |
| 6 | 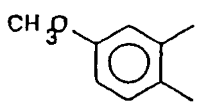 | 〃 | Colorless crystals<br>M.p. 178 - 179 °C |
| 7 | 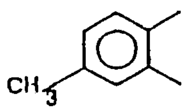 | 〃 | Colorless crystals<br>M.p. 199 - 200 °C |
| 8 | 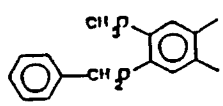 | 〃 | Colorless crystals<br>M.p. 172 - 174 °C |
| 9 | 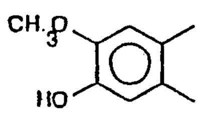 | 〃 | M.p. 231 °C (decomp.) |
| 10 | 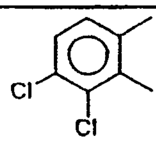 | 〃 | Colorless prisms<br>M.p. 209 - 210 °C |

| | | | |
|---|---|---|---|
| 11 | 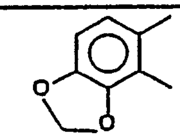 | $R^3 = -OCH_3$<br>$R^4 = -OCH_3$ | Pale yellow prisms<br>M.p. 228 - 229°C |
| 12 | 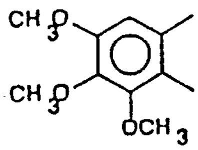 | $R^3 = -OC_2H_5$<br>$R^4 = -OC_2H_5$ | Colorless crystals<br>M.p. 138 - 140°C |
| 13 | " | $R^3 = -O\text{-}n\text{-}C_3H_7$<br>$R^4 = -O\text{-}n\text{-}C_3H_7$ | Colorless needles<br>M.p. 132°C |
| 14 | " | $R^3 = -OC_2H_5$<br>$R^4 = -OCH_3$ | Colorless needles<br>M.p. 158°C |
| 15 | " | $R^3 = -OCH_3$<br>$R^4 = -OC_2H_5$ | Colorless needles<br>M.p. 159°C |
| 16 | 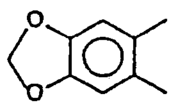 | $R^3 = -OC_2H_5$<br>$R^4 = -OC_2H_5$ | Colorless crystals<br>M.p. 158 - 159°C |
| 17 | " | $R^3 = -OCH_3$<br>$R^4 = -H$ | M.p. 152 - 154°C |
| 18 | " | $R^3 = -H$<br>$R^4 = -OCH_3$ | Colorless crystals<br>M.p. 169 - 171°C |

Table 4

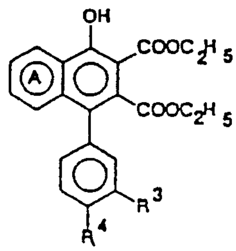

| Example Nos. | Ring A | $R^3$ / $R^4$ | Physical properties etc. |
|---|---|---|---|
| 19 | 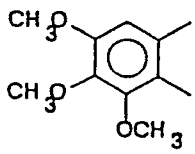 | $R^3$ = $-OCH_3$<br>$R^4$ = $-OCH_3$ | Colorless crystals<br>M.p. 138 - 140 °C |
| 20 | 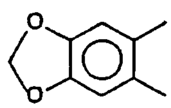 | $R^3$ = $-OC_2H_5$<br>$R^4$ = $-OC_2H_5$ | Colorless crystals<br>M.p. 150 - 151 °C |
| 21 | " | $R^3$ = $-O-i-C_3H_7$<br>$R^4$ = $-O-i-C_3H_7$ | M.p. 123 - 124 °C |

**Claims**

1. A process for preparing a naphthalene derivative of the formula:

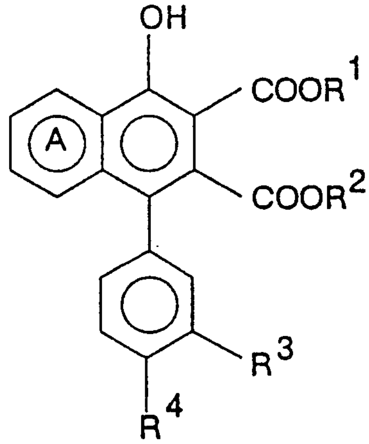

(I)

wherein each of $R^1$ and $R^2$ is a lower alkyl group; one of $R^3$ and $R^4$ is hydrogen atom or a lower alkoxy group and the other is a lower alkoxy group; and Ring A is a substituted or unsubstituted benzene ring, or a pharmaceutically acceptable salt thereof, which comprises reacting a benzaldehyde compound of the

formula:

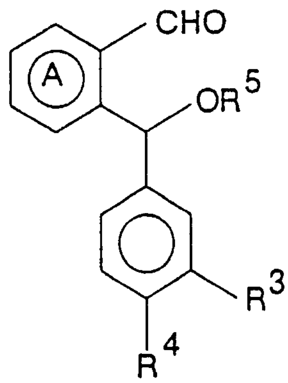

(II)

wherein $OR^5$ is a protected hydroxy group, and $R^3$, $R^4$ and Ring A are the same as defined above, or its di-lower alkyl acetal with an acetylene compound of the formula:

$$R^1\text{-O}\overset{O}{\overset{||}{C}}\text{-C}\equiv\text{C-}\overset{O}{\overset{||}{C}}\text{O-}R^2 \qquad (III)$$

wherein $R^1$ and $R^2$ are the same as defined above, and, if required, converting the product into a pharmaceutically acceptable salt thereof.

2. The process claimed in Claim 1, wherein Ring A is unsubstituted benzene ring, a benzene ring substituted with a lower alkylenedioxy group or a benzene ring substituted with one to 3 substituent(s) selected from a group consisting of a lower alkoxy group, a lower alkyl group, a phenyl-lower alkoxy group, hydroxy group and a halogen atom.

3. The process claimed in Claim 2, wherein Ring A is a benzene ring of the formula:

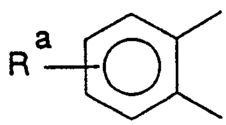 , 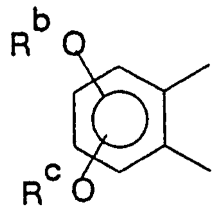 , 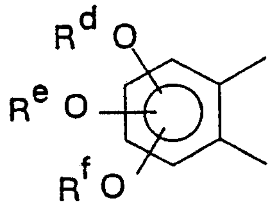

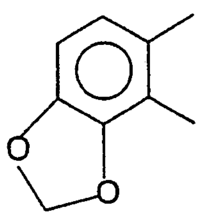 , 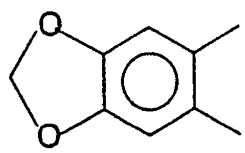 . or 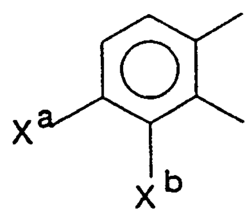

wherein $R^a$ is hydrogen atom, a lower alkyl group or a lower alkoxy group, $R^b$ is a lower alkyl group, $R^c$ is a lower alkyl group, a phenyl-lower alkyl group or hydrogen atom, each of $R^d$, $R^e$ and $R^f$ is a lower alkyl group and each of $X^a$ and $X^b$ is a halogen atom.

4. The process claimed in any of Claims 1 to 3, $OR^5$ is a lower alkoxy group or a lower alkanoyloxy group.

5. The process claimed in Claim 4, wherein each of $R^3$ and $R^4$ is a lower alkoxy group, and Ring A is a substituted benzene ring of the formula:

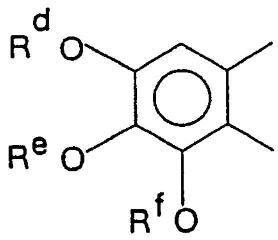

wherein each of $R^d$, $R^e$ and $R^f$ is a lower alkyl group.

6. The process claimed in Claim 5, wherein the naphthalene derivative is 1-(3,4-dimethoxyphenyl)-2,3-bis-(methoxycarbonyl )-4-hydroxy6,7,8-trimethoxynaphthalene or a pharmaceutically acceptable salt thereof.

7. The process according to any of Claims 1 through 6, wherein the reaction is carried out in the presence of an organic or inorganic acid at room temperature or under heating.

8. The process according to Claim 7, wherein the acid is selected from a group consisting of lower alkanoic acids, trihalo-lower alkanoic acids, benzenesuifonic acid, (lower alkylbenzene)sulfonic acids, lower alkylsulfonic acids, mineral acids, stannic halides, aluminum halides, titanium halides, zinc halides, ferric halides and boron halides-etherate.

9. The process according to Claim 8, wherein the reaction is carried out at a temperature between 50° C and 100° C.

10. A benzaldehyde compound of the formula:

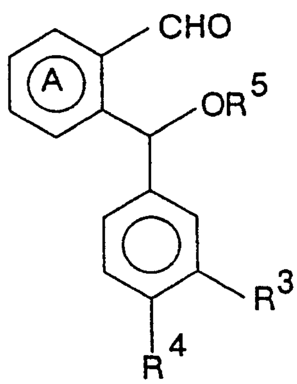

(II)

wherein one of $R^3$ and $R^4$ is hydrogen atom or a lower alkoxy group and the other is a lower alkoxy group, $OR^5$ is a protected hydroxy group, and Ring A is a substituted or unsubstituted benzene ring, or its di-lower alkylacetal.

11. The compound claimed in Claim 10, wherein Ring A is unsubstituted benzene ring, a benzene ring substituted with a lower alkylenedioxy group or a benzene ring substituted with one to 3 substituent(s) selected from a group consisting of a lower alkoxy group, a lower alkyl group, a phenyl-lower alkoxy group, hydroxy group and a halogen atom.

12. The compound claimed in Claim 11, wherein Ring A is a benzene ring of the formula: .

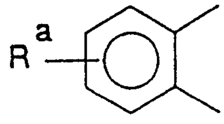,
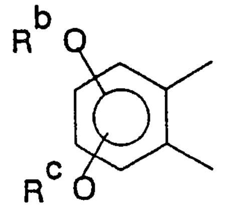,
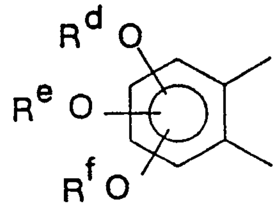

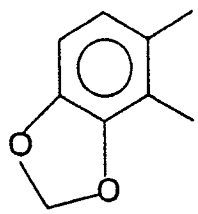,
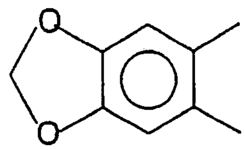
or
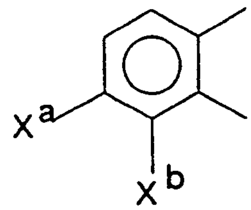

wherein $R^a$ is hydrogen atom, a lower alkyl group or a lower alkoxy group, $R^b$ is a lower alkyl group, $R^c$ is a lower alkyl group, a phenyl-lower alkyl group or hydrogen atom, each of $R^d$, $R^e$ and $R^f$ is a lower alkyl group and each of $X^a$ and $X^b$ is a halogen atom.

13. The compound claimed in Claim 12, wherein each of $R^3$ and $R^4$ is a lower alkoxy group, $OR^5$ is a lower alkoxy group or a lower alkanoyloxy group, and Ring A is a substituted benzene ring of the formula:

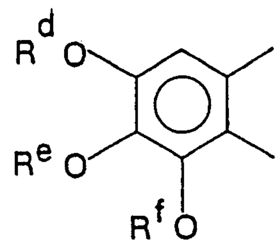

wherein each of $R^d$, $R^e$ and $R^f$ is a lower alkyl group.

Claims for the following Contracting States: ES,GR

1. A process for preparing a naphthalene derivative of the formula:

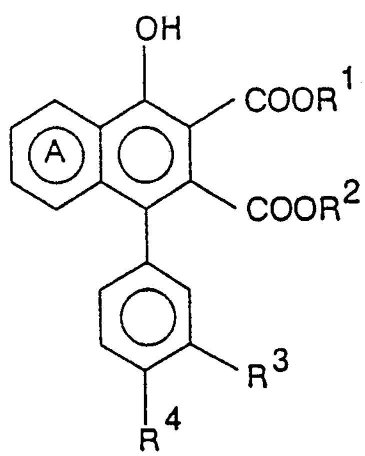
(I)

wherein each of $R^1$ and $R^2$ is a lower alkyl group; one of $R^3$ and $R^4$ is hydrogen atom or a lower alkoxy group and the other is a lower alkoxy group; and Ring A is a substituted or unsubstituted benzene ring, or a pharmaceutically acceptable salt thereof, which comprises reacting a benzaldehyde compound of the formula:

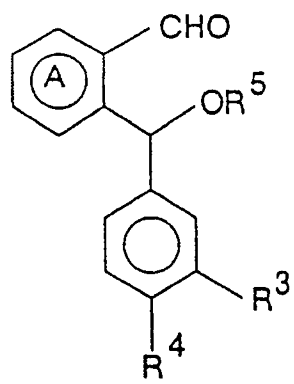

(II)

wherein $OR^5$ is a protected hydroxy group, and $R^3$, $R^4$ and Ring A are the same as defined above, or its di-lower alkyl acetal with an acetylene compound of the formula:

$$R^1\text{-O}\overset{O}{\overset{\|}{C}}\text{-C}\equiv\text{C-}\overset{O}{\overset{\|}{C}}\text{O-}R^2 \quad \text{(III)}$$

wherein $R^1$ and $R^2$ are the same as defined above, and, if required, converting the product into a pharmaceutically acceptable salt thereof.

2. The process claimed in Claim 1, wherein Ring A is unsubstituted benzene ring, a benzene ring substituted with a lower alkylenedioxy group or a benzene ring substituted with one to 3 substituent(s) selected from a group consisting of a lower alkoxy group, a lower alkyl group, a phenyl-lower alkoxy group, hydroxy group and a halogen atom.

3. The process claimed in Claim 2, wherein Ring A is a benzene ring of the formula:

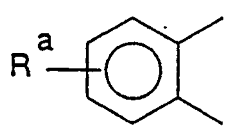 , 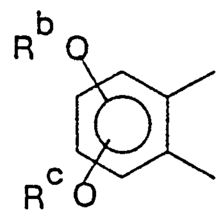 , 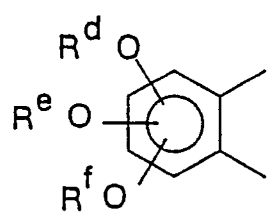

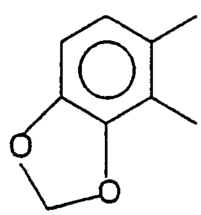 , 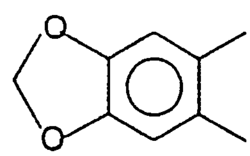 or 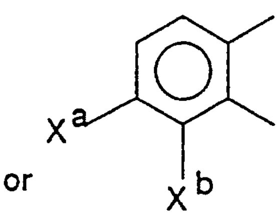

wherein $R^a$ is hydrogen atom, a lower alkyl group or a lower alkoxy group, $R^b$ is a lower alkyl group, $R^c$ is a lower alkyl group, a phenyl-lower alkyl group or hydrogen atom, each of $R^d$, $R^e$ and $R^f$ is a lower alkyl group and each of $X^a$ and $X^b$ is a halogen atom.

4. The process claimed in any of Claims 1 to 3, $OR^5$ is a lower alkoxy group or a lower alkanoyloxy group.

5. The process claimed in Claim 4, wherein each of $R^3$ and $R^4$ is a lower alkoxy group, and Ring A is a substituted benzene ring of the formula:

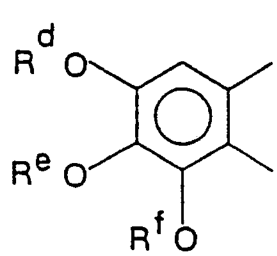

wherein each of $R^d$, $R^e$ and $R^f$ is a lower alkyl group.

6. The process claimed in Claim 5, wherein the naphthalene derivative is 1-(3,4-dimethoxyphenyl)-2,3-bis-(methoxycarbonyl)-4-hydroxy-6,7,8-trimethoxynaphthalene or a pharmaceutically acceptable salt thereof.

7. The process according to any of Claims 1 through 6, wherein the reaction is carried out in the presence of an organic or inorganic acid at room temperature or under heating.

8. The process according to Claim 7, wherein the acid is selected from a group consisting of lower alkanoic acids, trihalo-lower alkanoic acids, benzenesulfonic acid, (lower alkylbenzene)sulfonic acids, lower alkylsulfonic acids, mineral acids, stannic halides, aluminum halides , titanium halides, zinc halides, ferric halides and boron halides-etherate.

9. The process according to Claim 8, wherein the reaction is carried out at a temperature between 50° C and 100° C.

10. A process for the preparation of a benzaldehyde compound of the formula:

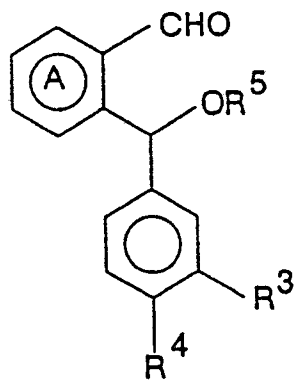

(II)

wherein one of $R^3$ and $R^4$ is hydrogen atom or a lower alkoxy group and the other is a lower alkoxy group, $OR^5$ is a protected hydroxy group, and Ring A is a substituted or unsubstituted benzene ring, or its di-lower alkylacetal, which comprises reacting the corresponding α-hydroxy-benzyl-benzaldehyde compound or its di-lower alkylacetal, with a compound of the formula $R^5X$, wherein X is a halide or the residue of a reactive derivative of an alkanoic acid, in the presence of an acid acceptor.

11. A process according to Claim 10, wherein Ring A is unsubstituted benzene ring, a benzene ring substituted with a lower alkylenedioxy group or a benzene ring substituted with one to 3 substituent(s) selected from a group consisting of a lower alkoxy group, a lower alkyl group, a phenyl-lower alkoxy group, hydroxy group and a halogen atom.

12. A process according to Claim 11, wherein Ring A is a benzene ring of the formula:

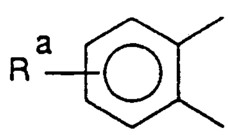 , 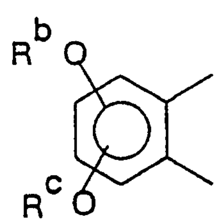 , 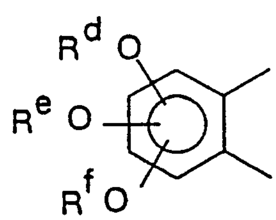

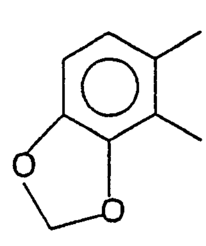 , 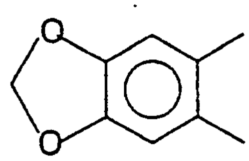 or 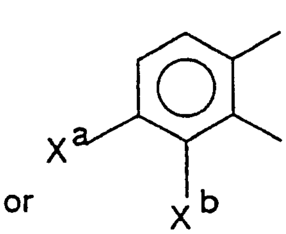

wherein $R^a$ is hydrogen atom, a lower alkyl group or a lower alkoxy group, $R^b$ is a lower alkyl group, $R^c$ is a lower alkyl group, a phenyl-lower alkyl group or hydrogen atom, each of $R^d$, $R^e$ and $R^f$ is a lower alkyl group and each of $X^a$ and $X^b$ is a halogen atom.

13. A process according to claim 12, wherein each of $R^3$ and $R^4$ is a lower alkoxy group, $OR^5$ is a lower alkoxy group or a lower alkanoyloxy group, and Ring A is a substituted benzene ring of the formula:

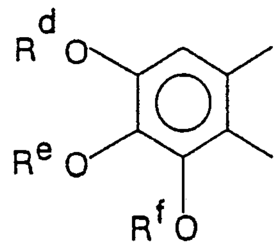

wherein each of $R^d$, $R^e$ and $R^f$ is a lower alkyl group.

European Patent Office

# EUROPEAN SEARCH REPORT

Application number

EP 90100832.6

**DOCUMENTS CONSIDERED TO BE RELEVANT**

| Category | Citation of document with indication, where appropriate, of relevant passages | Relevant to claim | CLASSIFICATION OF THE APPLICATION (Int Cl 5) |
|---|---|---|---|
| A | EP - A1 - 0 251 315 (TANABE SEITAKU) * Claims 13,14; example 1 * -- | 1,6-10 | C 07 C 69/94<br>C 07 D 317/60<br>C 07 C 67/347<br>C 07 C 47/575<br>C 07 D 317/54<br>C 07 C 69/157 |
| A | EP - A2 - 0 188 248 (TANABE SEITAKU) * Claim 35; example 1 * ---- | 1,6-10 | |

TECHNICAL FIELDS SEARCHED (Int Cl 5)

C 07 C 67/00
C 07 C 69/00
C 07 C 47/00

The present search report has been drawn up for all claims

| Place of search | Date of completion of the search | Examiner |
|---|---|---|
| VIENNA | 09-04-1990 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document